# EUROPEAN PATENT SPECIFICATION

(11) **EP 1 274 725 B1**
(45) Date of publication and mention of the grant of the patent: **07.06.2006**
(21) Application number: 01924610.7
(22) Date of filing: 03.04.2001
(51) Int. Cl.: C07K 14/005

(54) **USE OF HEV POLYPEPTIDES**
VERWENDUNG VON HEV-POLYPEPTIDEN
UTILISATION DES POLYPEPTIDES HEV

(30) Priority: 07.04.2000 US 195380 P
(43) Date of publication of application: 15.01.2003
(73) Proprietor: THE GOVERNMENT OF THE UNITED STATES OF AMERICA, as represented by the Secretary, Department of Health and Human Services, Atlanta, Georgia 30341 (US)
(72) Inventor: FIELDS, Howard, A., Marietta, GA 30068 (US); KHUDYAKOV, Yury, E., Duluth, GA 30096 (US); MENG, Jihong, Atlanta, GA 30306 (US)
(74) Representative: Leissler-Gerstl, Gabriele
(86) International application number: PCT/US2001/010696
(87) International publication number: WO 2001/077156

(56) References cited:
- WO-A-01/40270
- US-A- 6 022 685
- KHUDYAKOV ET AL: "Antigenic domains of the open reading frame 2-encoded protein of hepatitis E virus" JOURNAL OF CLINICAL MICROBIOLOGY, vol. 37, no. 9, September 1999 (1999-09), pages 2863-2871, XP002192675 cited in the application
- KHUDYAKOV ET AL: "Immunodominant antigenic regions in a structural protein of the hepatitis E virus" VIROLOGY, vol. 198, 1994, pages 390-393, XP001066315
- TSAREV ET AL: "Successful passive and active immunization of cynomolgus monkeys against hepatitis E" PROCEEDINGS OF THE NATIONAL ACADEMY SCIENCE, vol. 91, October 1994 (1994-10), pages 10198-10212, XP002192677 cited in the application
- The Third Annual Conference on Vaccine Research, April 30 - May 2, 2000, Renaissance Washington Hotel, Washington; Meng et al; Abstracts of submitted poster presentations - Poster P49, page 65) XP002192678

## Description

### FIELD OF THE INVENTION

The present invention relates to the fields of virology and immunology and provides immunogenic neutralizing hepatitis E virus (HEV) polypeptides for use as reagents for detecting HEV in a biological sample and for use as vaccines for the treatment or prophylaxis of HEV infection.

### BACKGROUND OF THE INVENTION

The disease caused by HEV is called hepatitis E, or enterically transmitted non-A non-B hepatitis (ET-NANBH). Other names include fecal-oral non-A non-B hepatitis, and A-like non-A non-B hepatitis. HEV is transmitted primarily by the fecal-oral route and causes epidemic or sporadic cases of hepatitis. Numerous HEV outbreaks have occurred in many developing countries, resulting in tens of thousands of people being infected. The mortality rate from acute HEV infection ranges from 0.5% to 1% for the general population to as high as 20% for infected pregnant women. Although only a few cases have been diagnosed in industrialized countries, anti-HEV antibodies have been found in a significant proportion of blood donors or healthy individuals. The reason for this relatively high seroprevalence is not yet well explained but may be associated with the zoonotic feature of the HEV infection.

HEV is a non-enveloped virus. The viral genome consists of three discontinuous, partially overlapping open reading frames (ORFs), with ORF1 encoding non-structural proteins (pORF1), ORF2 encoding the putative 660 amino acid (aa) long capsid protein (pORF2), and ORF3 encoding a small protein (pORF3) of unknown function. The nucleotide and amino acid sequences of ORF2 and pORF2 are shown in SEQ. ID NO.:1. The full-length genomes of several HEV strains from North America and Asia have been sequenced. Nucleotide sequence alignments and phylogenetic analysis based on the full-length sequences suggest the presence of three distinct genotypes, represented by Burma, Mexico, and US strains. More genotypes or substrains are being counted by means of partial sequence comparisons.

Recombinant proteins spanning the sequence of amino acids 112 through 660 or amino acids 225 through 660 of pORF2 induced protective immune responses in non-human primate animals (Purdy et al., 1993; Tsarev et al., 1994, 1997; Yarbough et al., 1997). Recently, six antigenic domains with 26 IgG antibody-reactive epitopes and 24 IgM antibody-reactive epitopes were identified within pORF2 by using three sets of overlapping 18-mer, 25-mer, and 30-mer synthetic peptides (Khudyakov et al., 1999). Antibodies against HEV recombinant C2 protein (Purdy et al., 1992), which contains the carboxyl-end two thirds of the HEV Burma pORF2, efficiently neutralized the HEV Burma, Mexico and Pakistan strains in an *in vitro* neutralization assay (Meng et al., 1998).

The antigenic characteristics of HEV have not been thoroughly investigated, however, although several antigenic regions have been found within pORF1, pORF2, and pORF3. In particular, HEV neutralizing polypeptides had not yet been identified.

Viral neutralizing antigenic epitopes have the unique functional property to cause loss of virus infectivity when an antibody elicited to the epitope binds to a virus particle. Identification of such an epitope on the surface of a virion is of importance for studies on the mechanisms of neutralization, as a foundation for understanding the molecular basis of serotyping, and as a starting point for developing subunit vaccines.

Short synthetic peptides have been reported to elicit neutralizing antibodies to hepatitis A virus (Emini et al., 1985), hepatitis B virus (Neurath et al., 1986), and hepatitis C virus (Shimizu et al., 1996). However, short synthetic peptides only form linear epitopes which have low intrinsic immunogenicity due to their inability to elicit T-cell responses. If they are administered in a manner that enables delivery of T-cell help, they can elicit reasonably robust antibody responses. Antibodies raised in this manner, however, while reacting well with the immunogen and unfolded full-length protein, usually bind to native antigens with such a low affinity that they do not exhibit biological activity. Thus, with a few notable exceptions, the use of a synthetic peptide to elicit a neutralizing antibody response to a virus has proven to be a great disappointment (Yewdell & Bennink, 1997).

The development of an inactivated or live attentuated HEV vaccine has been hampered by the lack of an efficient cell culture system capable of supporting HEV replication and propagation. Identification of the neutralizing antigenic epitope(s) is an urgent need to develop a safe and effective subunit vaccine for control of HEV infection.

Therefore, a long-felt and desperate need exists for the identification of neutralizing immunogenic HEV polypeptides that can be used in a clinical setting as an HEV vaccine and thereby to protect against HEV infection. In addition, there is a need for neutralizing immunogenic HEV polypeptides in both clinical and laboratory settings to study HEV infection and how it affects the host Moreover, a polypeptide that models the neutralizing epitope of HEV would be useful.

### SUMMARY OF THE INVENTION

Immunogenic HEV polypeptides and methods of use are provided. The polypeptide is an isolated, recombinant, or synthetic polypeptide containing at least 50 amino acid residues between amino acid residues 452 through 617 from the C-terminal of the HEV pORP2 protein, including at least one neutralizing epitope. Most preferably, a polypeptide includes amino acid residues 452 through 617 of the C-terminal of the HEV pORF2 protein.

Each polypeptide is useful, alone or combination with other polypeptides described herein, as a reagent for studying the pathogenesis of HEV. In particular, the reagent can be used to identify the types of immune responses that confer protection against HEV infection or reduce progression of the disease. In addition, the polypeptides are useful as reagents for monitoring drug efficacy in clinical trials or treatment regimens in patients who are undergoing HEV therapy. The polypeptides are also useful to model the neutralizing antigenic epitope(s) of HEV.

One or more of the polypeptides are also useful as a vaccine composition when combined with a pharmaceutical carrier for the prophylaxis, treatment, or prevention of HEV infection. The vaccine composition is administered to an individual prior to HEV exposure to minimize or prevent HEV infection or is administered after a patient has been infected to reduce the severity of infection and retard or halt progression of the disease.

Antibodies to the neutralizing polypeptides are provided. Neutralizing antibodies, preferably monoclonal antibodies, are also provided.

It is therefore an object of the present invention to provide an immunogenic polypeptide that reacts with antibodies, T helper lymphocytes, or T cytotoxic lymphocytes from HEV-positive patients.

It is a further object of the invention to provide neutralizing antigenic epitopes of HEV.

It is a further object to provide neutralizing antibodies, and particularly monoclonal antibodies, to HEV.

It is a further object of the present invention to provide a vaccine for the prevention or treatment of HEV infection.

It is a further object of the present invention to provide an HEV vaccine that confers protection against a wide variety of HEV strains and variants.

It is a further object of the present invention to provide a safe HEV vaccine that cannot revert to a pathogenic state.

It is a further object of the present invention to provide a research tool or reagent to study HEV pathogenesis.

It is a further object of the present invention to provide a research tool or reagent to monitor drug efficacy in clinical trials or treatment regimens.

Other features, objects, and advantages of the invention and its preferred embodiments will become apparent from the detailed description which follows.

### DETAILED DESCRIPTION OF THE INVENTION

Immunogenic HEV polypeptides and methods of use are provided. The polypeptide is an isolated, recombinant, or synthetic polypeptide containing at least 50 amino acid residue between amino acid residues 452 through 617 from the C-terminal of the HEV pORF2 protein, including at least one neutralizing epitope. Most preferably, a polypeptide includes amino acid residues 452 through 617 of the C-terminal of the HEV pORF2 protein.

Each polypeptide is useful, alone or combination with other polypeptides described herein, as a reagent for studying the pathogenesis of HEV. In particular, the reagent can be used to identify the types of immune responses that confer protection against BEV infection or reduce progression of the disease. In addition, the polypeptides are useful as reagents for monitoring drug efficacy in clinical trials or treatment regimens in patients who are undergoing HEV therapy. The polypeptides are also useful to model the neutralizing antigenic epitope(s) of HEV.

One or more of the polypeptides are also useful as a vaccine composition when combined with a pharmaceutical carrier for the prophylaxis, treatment, or prevention of HEV infection. The vaccine composition is administered to an individual prior to HEV exposure to minimize or prevent HEV infection or is administered after a patient has been infected to reduce the severity of infection and retard or halt progression of the disease.

Antibodies to the neutralizing polypeptides are provided. Neutralizing antibodies, preferably monoclonal antibodies, are also provided.

### Definitions

The terms "a", "an" and "the" as used herein are defined to mean "one or more" and include the plural unless the context is inappropriate.

By "isolated" is meant peptide free from at least some of the components with which it naturally occurs.

"Peptides," "polypeptides", and "oligopeptides" are used interchangeably and are defined herein as chains of amino acids (typically L-amino acids) in which carbons are linked through peptide bonds formed by a condensation reaction between the carboxyl group of the carbon of one amino acid and the amino group of the carbon of another amino acid. The terminal amino acid at one end of the chain (*i.e*., the amino terminal) has a free amino group, while the terminal amino acid at the other end of the chain (*i.e.,* the carboxy terminal) has a free carboxyl group.

Typically, the amino acids making up a peptide are numbered in order, starting at the amino terminal and increasing in the direction of the carboxy terminal of the peptide. Thus, when one amino acid is said to "follow" another, that amino acid is positioned closer to the carboxy terminal of the peptide than the "preceding" amino acid.

The term "residue" is used herein to refer to an amino acid (D or L) or an amino acid mimetic that is incorporated into a oligopeptide by an amide bond or an amide bond mimetic. As such, the amino acid may be a naturally occurring amino acid or, unless otherwise limited, may encompass known analogs of natural amino acids that function in a manner similar to the naturally occurring amino acids (*i.e.* amino acid mimetics). Moreover, an amide bond mimetic includes peptide backbone modifications well known to those skilled in the art.

"Antigen" refers to an entity or fragment thereof which can induce an immune response in a mammal. The term includes immunogens and regions responsible for antigenicity or antigenic determinants.

"Neutralizing antibody" refers to an antibody that blocks the attachment of HEV to a cell.

"Neutralizing antigenic epitope" or "neutralizing epitope" refers to an epitope that elicits a neutralizing antibody.

"Antigenic determinant" refers to a region of a protein recognized by an antibody or T cell receptor, *e.g.,* in serum raised against wild-type protein.

The phrases "specifically binds to a peptide" or "specifically immunoreactive with", when referring to an antibody or T cell receptor, refers to a binding reaction which is determinative of the presence of the peptide, or an antibody or T cell receptor to the peptide, in the presence of a heterogeneous population of proteins and other biologics. Thus, under designated immunoassay conditions, the specified antibodies or T cell receptors bind preferentially to a particular peptide and do not bind in a significant amount to other proteins present in the sample. Specific binding to a peptide under such conditions requires an antibody or T cell that is selected for its specificity for a particular protein. A variety of immunoassay formats may be used to select antibodies specifically immunoreactive with a particular protein. For example, solid phase ELISA immunoassays are routinely used to select monoclonal antibodies specifically immunoreactive with a protein. *See,* Harlow and Lane (1988) *Antibodies, A Laboratory Manual,* Cold Spring Harbor Publications, New York, for a description of immunoassay formats and conditions that can be used to determine specific immunoreactivity.

"Conservative variations" or "conservative modified variations" of a particular sequence refers to amino acids encoded by nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given peptide. Such nucleic acid variations are silent variations, which are one species of conservatively modified variations. One of skill will recognize that each codon in a nucleic acid (except AUG, which is ordinarily the only codon for methionine) can be modified to yield a functionally identical molecule by standard techniques. Accordingly, each silent variation of a nucleic acid which encodes a peptide is implicit in any described amino acid sequence. Furthermore, one of skill will recognize that individual substitutions, deletions or additions which alter, add or delete a single amino acid or a small percentage of amino acids (typically less than 5%, more typically less than 1%) in an encoded sequence are conservatively modified variations where the alterations result in the substitution of an amino acid with a chemically similar amino acid. Conservative substitution tables providing functionally similar amino acids are well known in the art. The following six groups each contain amino acids that are conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

Two polypeptides are said to be "identical" if the sequence of amino acid residues in the two sequences is the same when aligned for maximum correspondence. Optimal alignment of sequences for comparison may be conducted by the local homology algorithm of Smith and Waterman Adv. *Appl. Math.* 2: 482 (1981), by the homology alignment algorithm of Needleman and Wunsch *J. Mol. Biol.* 48:443 (1970), by the search for similarity method of Pearson and Lipman *Proc. Natl. Acad. Sci. (U.S.A.)* 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The term "substantial identity" means that a polypeptide comprises a sequence that has at least 85% sequence identity or homology, preferably 90%, more preferably 95% or more, compared to a reference sequence over a comparison window of about 10 to about 20 amino acids. Another indication that polypeptide sequences are substantially identical is if one peptide is immunologically reactive with antibodies raised against the disclosed peptide. Thus, the peptides for the use of the invention include peptides immunologically reactive with antibodies raised against the disclose immunogenic peptides.

### Synthetic Polypeptides

The polypeptides described herein generally contain from 50 to about 166 amino acid residues, more preferably, from about 100 to about 166 amino acid residues and, even more preferably, about 166 amino acid residues. The polypeptides can be prepared using any of a number of chemical peptide synthesis techniques well known to those of ordinary skill in the art including both solution methods and solid phase methods, with solid phase synthesis being presently preferred.

In particular, solid phase synthesis in which the C-terminal amino acid of the polypeptide sequence is attached to an insoluble support followed by sequential addition of the remaining amino acids in the sequence is a preferred synthetic method for preparing the polypeptides. Techniques for solid phase synthesis are described by Merrifield, *et al., J. Am. Chem. Soc. 85*:2149-2156 (1963). Many automated systems for performing solid phase peptide synthesis are commercially available.

Solid phase synthesis is started from the carboxy-terminal end (*i.e.,* the C-terminus) of the polypeptide by coupling a protected amino acid via its carboxyl group to a suitable solid support. The solid support used is not a critical feature provided that it is capable of binding to the carboxyl group while remaining substantially inert to the reagents utilized in the peptide synthesis procedure. For example, a starting material can be prepared by attaching an amino-protected amino acid via a benzyl ester linkage to a chloromethylated resin or a hydroxymethyl resin or via an amide bond to a benzhydrylamine (BHA) resin or p-methylbenzhydrylamine (MBHA) resin. Materials suitable for use as solid supports are well known to those of skill in the art and include, but are not limited to, the following: halomethyl resins, such as chloromethyl resin or bromomethyl resin; hydroxymethyl resins; phenol resins, such as 4-(a-[2,4-dimethoxyphenyl]-Fmoc-aminomethyl)phenoxy resin; tert-alkyloxycarbonyl-hydrazidated resins; and the like. Such resins are commercially available and their methods of preparation are known to those of ordinary skill in the art.

The acid form of the peptides may be prepared by the solid phase peptide synthesis procedure using a benzyl ester resin as a solid support. The corresponding amides may be produced by using benzhydrylamine or methylbenzhydrylamine resin as the solid support. Those skilled in the art will recognize that when the BHA or MBHA resin is used, treatment with anhydrous hydrofluoric acid to cleave the peptide from the solid support produces a peptide having a terminal amide group.

The α-amino group of each amino acid used in the synthesis should be protected during the coupling reaction to prevent side reactions involving the reactive α-amino function. Certain amino acids also contain reactive side-chain functional groups (*e.g.* sulfhydryl, amino, carboxyl, hydroxyl, *etc.*) which must also be protected with appropriate protecting groups to prevent chemical reactions from occurring at those sites during the peptide synthesis. Protecting groups are well known to those of skill in the art. *See,* for example, *The Peptides: Analysis, Synthesis, Biology, Vol 3: Protection of Funcfional Groups in Peptide Synthesis* (Gross and Meienhofer (eds.), Academic Press, N.Y. (1981)).

A properly selected α-amino protecting group will render the α-amino function inert during the coupling reaction, will be readily removable after coupling under conditions that will not remove side chain protecting groups, will not alter the structure of the peptide fragment, and will prevent racemization upon activation immediately prior to coupling. Similarly, side-chain protecting groups must be chosen to render the side chain functional group inert during the synthesis, must be stable under the conditions used to remove the α-amino protecting group, and must be removable after completion of the peptide synthesis under conditions that will not alter the structure of the peptide.

Coupling of the amino acids may be accomplished by a variety of techniques known to those of skill in the art. Typical approaches involve either the conversion of the amino acid to a derivative that will render the carboxyl group more susceptible to reaction with the free N-terminal amino group of the peptide fragment, or use of a suitable coupling agent such as, for example, N,N'-dicyclohexylcarbodimide (DCC) or N,N'-diisopropylcarbodiimide (DIPCDI). Frequently, hydroxybenzotriazole (HOBt) is employed as a catalyst in these coupling reactions.

Generally, synthesis of the peptide is commenced by first coupling the C-terminal amino acid, which is protected at the N-amino position by a protecting group such as fluorenylmethyloxycarbonyl (Fmoc), to a solid support. Prior to coupling of Fmoc-Asn, the Fmoc residue has to be removed from the polymer. Fmoc-Asn can, for example, be coupled to the 4-(a-[2,4-dimethoxyphenyl]-Fmoc-amino-methyl)phenoxy resin using N,N'-dicyclohexylcarbodimide (DCC) and hydroxybenzotriazole (HOBt) at about 25 C for about two hours with stirring. Following the coupling of the Fmoc-protected amino acid to the resin support, the α-amino protecting group is removed using 20% piperidine in DMF at room temperature.

After removal of the α-amino protecting group, the remaining Fmoc-protected amino acids are coupled stepwise in the desired order. Appropriately protected amino acids are commercially available from a number of suppliers (*e.g*., Novartis (Switzerland) or Bachem (California)). As an alternative to the stepwise addition of individual amino acids, appropriately protected peptide fragments consisting of more than one amino acid may also be coupled to the "growing" peptide. Selection of an appropriate coupling reagent, as explained above, is well known to those of skill in the art.

Each protected amino acid or amino acid sequence is introduced into the solid phase reactor in excess and the coupling is carried out in a medium of dimethylformamide (DMF), methylene chloride (CH₂Cl₂), or mixtures thereof. If coupling is incomplete, the coupling reaction may be repeated before deprotection of the N-amino group and addition of the next amino acid. Coupling efficiency may be monitored by a number of means well known to those of skill in the art. A preferred method of monitoring coupling efficiency is by the ninhydrin reaction. Peptide synthesis reactions may be performed automatically using a number of commercially available peptide synthesizers such as the Biosearch 9500™ synthesizer, Biosearch, San Raphael, CA).

The peptide can be cleaved and the protecting groups removed by stirring the insoluble carrier or solid support in anhydrous, liquid hydrogen fluoride (HF) in the presence of anisole and dimethylsulfide at about 0 C for about 20 to 90 minutes, preferably 60 minutes; by bubbling hydrogen bromide (HBr) continuously through a 1 mg/10 mL suspension of the resin in trifluoroacetic acid (TFA) for 60 to 360 minutes at about room temperature, depending on the protecting groups selected; or by incubating the solid support inside the reaction column used for the solid phase synthesis with 90% trifluoroacetic acid, 5% water and 5% triethylsilane for about 30 to 60 minutes. Other deprotection methods well known to those of skill in the art may also be used.

The peptides can be isolated and purified from the reaction mixture by means of peptide purification well known to those of skill in the art. For example, the peptides may be purified using known chromatographic procedures such as reverse phase HPLC, gel permeation, ion exchange, size exclusion, affinity, partition, or countercurrent distribution.

### Recombinant Polypeptides

It will be understood by those of ordinary skill in the art that the polypeptides can also be prepared by other means including, for example, recombinant techniques. Examples of appropriate cloning and sequencing techniques, and instructions sufficient to direct persons of skill through many cloning exercises are found in Sambrook *et al.* (1989) *Molecular Cloning - A Laboratory Manual* (2nd ed.) Vol. 1-3, Cold Spring Harbor Laboratory, Cold Spring Harbor Press, NY, (Sambrook). Product information from manufacturers of biological reagents and experimental equipment, such as the SIGMA Chemical Company (Saint Louis, MO), also provide information useful in known biological methods.

The polypeptides described herein are derived from pORF2 protein. The nucleotide sequence of the nucleic acid that encodes pORF2 is known. Accordingly, the known nucleic acid sequence can be used to make the polypeptides recombinantly or a nucleic acid encoding the desired polypeptide can be derived from the amino acid sequence.

Generally, this involves creating a nucleic acid sequence that encodes the polypeptide, placing the nucleic acid in an expression cassette under the control of a particular promoter, expressing the polypeptide in a host, isolating the expressed polypeptide and, if required, renaturing the polypeptide. Techniques sufficient to guide one of skill through such procedures are found in Sambrook, *supra.*

Provided with the polypeptide sequences described herein, one of skill will recognize a variety of equivalent nucleic acids that encode the polypeptide. This is because the genetic code requires that each amino acid residue in a peptide is specified by at least one triplet of nucleotides in a nucleic acid which encodes the peptide. Due to the degeneracy of the genetic code, many amino acids are equivalently coded by more than one triplet of nucleotides. For instance, the triplets CGU, CGC, CGA, CGG, AGA, and AGG all encode the amino acid arginine. Thus, at every position where an arginine is to be encoded by a nucleic acid triplet, the nucleic acid has any of the triplets which encode arginine. One of skill is thoroughly familiar with the genetic code and its use. An introduction to the subject is found in, for example, chapter 15 of Watson, *et al., Molecular Biology of the Gene* (Fourth Edition, The Benjamin/Cummings Company, Inc., Menlo Park, California (1987)), and the references cited therein.

Although any nucleic acid triplet or codon which encodes an amino acid can be used to specify the position of the amino acid in a peptide, certain codons are preferred. It is desirable to select codons for elevated expression of an encoded peptide, for example, when the peptide is purified for use as an immunogenic reagent. Codons are selected by reference to species codon bias tables, which show which codons are most typically used by the organism in which the peptide is to be expressed. The codons used frequently by an organism are translated by the more abundant t-RNAs in the cells of the organism. Because the t-RNAs are abundant, translation of the nucleic acid into a peptide by the cellular translation machinery is facilitated. Codon bias tables are available for most organisms. For an introduction to codon bias tables, *see, e.g.* Watson *et al., supra.*

### Conservative Substitutions

In addition, it will be readily apparent to those of ordinary skill in the art that the polypeptides described herein and the nucleic acid molecules encoding such immunogenic polypeptides can be subject to various conservative changes, such as insertions, deletions, and substitutions, where such changes might provide for certain advantages in their use, *i.e.,* to increase biological activity.

One of skill will appreciate that many conservative variations of nucleic acid constructs yield a functionally identical construct. For example, due to the degeneracy of the genetic code, silent substitutions (*i.e*, substitutions of a nucleic acid sequence which do not result in an alteration in an encoded peptide) are an implied feature of *every* nucleic acid sequence which encodes an amino acid. In addition, one of skill will recognize many ways of generating alterations in a given nucleic acid construct. Such well-known methods include site-directed mutagenesis, PCR amplification using degenerate oligonucleotides, exposure of cells containing the nucleic acid to mutagenic agents or radiation, chemical synthesis of a desired oligonucleotide (*e.g.,* in conjunction with ligation and/ or cloning to generate large nucleic acids) and other well-known techniques. *See,* Giliman and Smith (1979) *Gene* 8:81-97, Roberts *et al.* (1987) *Nature* 328:731-734, and Sambrook, *supra.*

Modifications to nucleic acids are evaluated by routine screening techniques in suitable assays for the desired characteristic. For instance, changes in the immunological character of encoded peptides can be detected by an appropriate immunological assay. Modifications of other properties such as nucleic acid hybridization to a complementary nucleic acid, redox or thermal stability of encoded proteins, hydrophobicity, susceptibility to proteolysis, or the tendency to aggregate are all assayed according to standard techniques.

Similarly, conservative amino acid substitutions, in one or a few amino acids in an amino acid sequence of a protein are substituted with different amino acids with highly similar properties (*see*, the definitions section, *supra*), are also readily identified as being highly similar to a disclosed construct.

### Immunogenic Conjugates

Immunogenic conjugates containing one or more of the synthetic or recombinant polypeptides described above, covalently attached to a carrier protein, are also provided. Suitable carrier proteins include, but are not limited to, the following: thyroglobulin, albumins such as human serum albumin, tetanus toxoid, polyamino acids such as poly(D-lysine:D-glutamic acid), influenza, hepatitis B virus core protein, hepatitis B virus recombinant vaccine, and the like.

When the polypeptide and carrier protein are relatively short in length, they can be synthesized using standard chemical peptide synthesis techniques. When both molecules are relatively short, a chimeric molecule is optionally synthesized as a single contiguous polypeptide. Alternatively, the peptide and the carrier molecule can be synthesized separately and then fused chemically. Alternatively, the polypeptide and carrier can be produced individually recombinantly and then fused chemically. Most preferably, the polypeptide and carrier are produced recombinantly as a single polypeptide.

Generally, this involves creating a nucleic acid sequence that encodes the polypeptide-carrier protein immunogenic conjugate, placing the nucleic acid in an expression cassette under the control of a particular promoter, expressing the protein in a host, isolating the expressed protein and, if required, renaturing the protein. Techniques sufficient to guide one of skill through such procedures are found in Sambrook, *supra.*

While the polypeptide and carrier molecule are often joined directly together, one of skill will appreciate that the molecules may be separated by a spacer molecule (*e.g*., a peptide) consisting of one or more amino acids. Generally, the spacer will have no specific biological activity other than to join the immunogenic peptide to the carrier protein, or to preserve some minimum distance or other spatial relationship between them. However, the constituent amino acids of the spacer may be selected to influence some property of the molecule such as the folding, net charge, or hydrophobicity.

Once expressed, recombinant immunogenic conjugates can be purified according to standard procedures, including ammonium sulfate precipitation, affinity columns, column chromatography, gel electrophoresis and the like. Substantially pure compositions of about 50 to 95% homogeneity are preferred, and 80 to 95% or greater homogeneity are most preferred for use as therapeutic agents.

One of skill in the art will recognize that after chemical synthesis, or recombinant expression, the immunogenic conjugates of the present invention may possess a conformation substantially different than the native conformations of the constituent polypeptides. In this case, it is often necessary to denature and reduce the polypeptide and then to cause the polypeptide to re-fold into the preferred conformation. Methods of reducing and denaturing proteins and inducing re-folding are well known to those of skill in the art.

### Multiepitope Polypeptides

In an alternative embodiment, the immunogenic polypeptides described herein are combined into multiepitope, or polyepitope, polypeptides or proteins. Typically, 2 to 12 of the immunogenic polypeptides are fused into a single polypeptide by recombinant or synthetic techniques.

In recombinant procedures, multiepitope proteins are made by ligating synthetic or recombinant nucleic acids which encode immunogenic peptides. These nucleic acids are ligated enzymatically (*e.g*., using a DNA ligase enzyme) or synthetically. Alternatively, a single nucleic acid molecule is synthesized which encodes multiple immunogenic peptides. In either case, the resulting nucleic acid encodes multiple immunogenic peptides, all in the same reading frame. Thus, the translated polypeptide contains two or more immunogenic peptide domains.

When the multiepitope polypeptides are produced by automated chemical synthetic procedures, concatamers of peptides are coupled directly. This is performed chemically by joining peptides using standard chemical methods. Alternatively, a polypeptide is synthetically produced that encodes multiple immunogenic peptides.

Chemical or recombinant linker regions are optionally included between immunogenic polypeptide domains to facilitate presentation of the domains to antibodies which bind the domains. In preferred embodiments, 10 to 50 amino acids are inserted between immunogenic domains. Essentially any amino acid or chemical moiety which forms amide and carboxyl linkages can be used as a linker.

### Antibody Production

Antibodies that bind with specificity to the polypeptides described above are also provided. The antibodies include individual, allelic, strain, or species variants, and fragments thereof, both in their naturally occurring (full-length) forms and in recombinant forms. Additionally, antibodies are raised to these polypeptides in either their native configurations or in non-native configurations. Anti-idiotypic antibodies can also be generated. Many methods of making antibodies are known to persons of skill. The antibodies are useful as research tools for the isolation of additional quantities of the antigenic polypeptides and for studying the pathogenesis of HEV in general. The antibodies may also be useful therapeutically for passive immunization of an HEV-infected patient

The antibodies include neutralization antibodies. Methods for screening antibodies for neutralization are known. A specific *in vitro* neutralization assay is described in Meng et al., 1997; 1998 and below.

The following discussion is presented as a general overview of the techniques available for the production of antibodies; however, one of skill will recognize that many variations upon the following methods are known.

A number of immunogens are used to produce antibodies specifically reactive with polypeptides. Recombinant or synthetic polypeptides of fifty amino acids in length, or greater, selected from the polypeptides disclosed herein are the preferred polypeptide immunogens for the production of monoclonal or polyclonal antibodies. In one class of preferred embodiments, an immunogenic polypeptide conjugate is also included as an immunogen. The polypeptides are used either in pure, partially pure or impure form.

Recombinant polypeptides are expressed in eukaryotic or prokaryotic cells and purified using standard techniques. The polypeptide, or a synthetic version thereof, is then injected into an animal capable of producing antibodies. Either monoclonal or polyclonal antibodies can be generated for subsequent use in immunoassays to measure the presence and quantity of the polypeptide.

Methods of producing polyclonal antibodies are known to those of skill in the art. In brief, an immunogen, preferably a purified peptide, a peptide coupled to an appropriate carrier (*e.g.,* GST, keyhole limpet hemanocyanin, *etc*.), or a peptide incorporated into an immunization vector such as a recombinant vaccinia virus is mixed with an adjuvant and animals are immunized with the mixture. The animal's immune response to the immunogen preparation is monitored by taking test bleeds and determining the titer of reactivity to the peptide of interest. When appropriately high titers of antibody to the immunogen are obtained, blood is collected from the animal and antisera are prepared. Further fractionation of the antisera to enrich for antibodies reactive to the peptide is performed where desired.

Antibodies, including binding fragments and single chain recombinant versions thereof, against the polypeptides are raised by immunizing animals, *e.g.,* using immunogenic conjugates comprising a polypeptide covalently attached (conjugated) to a carrier protein as described above. The immunogen of interest is a polypeptide of at least 50 amino acids, in another embodiment the polypeptide is 100 amino acids in length, and in another embodiment, the fragment is about 166 amino acids in length and comprises amino acids acid residues 452 and 617 from the C-terminal of the HEV pORF2 protein. The immunogenic conjugates are typically prepared by coupling the polypeptide to a carrier protein (*e.g.*, as a fusion protein) or, alternatively, they are recombinantly expressed in an immunization vector.

Monoclonal antibodies are prepared from cells secreting the desired antibody. These antibodies are screened for binding to normal or modified peptides, or screened for agonistic or antagonistic activity. Specific monoclonal and polyclonal antibodies will usually bind with a K_{D} of at least about 0.1 mM, more usually at least about 50 mM, and most preferably at least about 1 mM or better. Often, specific monoclonal antibodies bind with a K_{D} of 0.1 mM or better.

In some instances, it is desirable to prepare monoclonal antibodies from various mammalian hosts, such as mice, rodents, primates, humans, *etc.* Description of techniques for preparing such monoclonal antibodies are found in Kohler and Milstein (1975) *Nature* 256: 495-497. Summarized briefly, this method proceeds by injecting an animal with an immunogen, *i.e.,* an immunogenic peptide of the present invention either alone or optionally linked to a carrier protein. The animal is then sacrificed and cells taken from its spleen, which are fused with myeloma cells. The result is a hybrid cell or "hybridoma" that is capable of reproducing *in vitro.* The population of hybridomas is then screened to isolate individual clones, each of which secrete a single antibody species to the immunogen In this manner, the individual antibody species obtained are the products of immortalized and cloned single B cells from the immune animal generated in response to a specific site recognized on the immunogenic substance.

Alternative methods of immortalization include transformation with Epstein Barr Virus, oncogenes, or retroviruses, or other methods known in the art. Colonies arising from single immortalized cells are screened for production of antibodies of the desired specificity and affinity for the antigen, and yield of the monoclonal antibodies produced by such cells is enhanced by various techniques, including injection into the peritoneal cavity of a vertebrate (preferably mammalian) host. The polypeptides and antibodies of the present invention are used with or without modification, and include chimeric antibodies such as humanized murine antibodies. Other suitable techniques involve selection of libraries of recombinant antibodies in phage or similar vectors. See, *Huse et al.* (1989) *Science* 246:1275-1281; and Ward *et al.* (1989) *Nature*341: 544-546.

Frequently, the polypeptide or antibody will be labeled by joining, either covalently or non covalently, a substance which provides for a detectable signal. A wide variety of labels and conjugation techniques are known and are reported extensively in both the scientific and patent literature. Suitable labels include radionucleotides, enzymes, substrates, cofactors, inhibitors, fluorescent moieties, chemiluminescent moieties, magnetic particles, and the like. Patents teaching the use of such labels include U.S. Patent Nos. 3,817,837; 3,850,752; 3,939,350; 3,996,345; 4,277,437; 4,275,149; and 4,366,241.

As mentioned above, the antibodies provided herein can be used in affinity chromatography for isolating additional amounts of the polypeptides identified herein. Columns are prepared, *e.g.,* with the antibodies linked to a solid support, *e.g.,* particles, such as agarose, Sephadex, or the like, where a cell lysate is passed through the column, washed, and treated with increasing concentrations of a mild denaturant, whereby purified polypeptides are released. In addition, the antibodies can be used to screen expression libraries for particular expression products, for example, HEV proteins. Usually, the antibodies in such a procedure are labeled with a moiety allowing easy detection of presence of antigen by antibody binding. Moreover, antibodies raised against the immunogenic polypeptides described herein can also be used to raise anti-idiotypic antibodies. Such antibodies are useful for detecting or diagnosing various pathological or resistance conditions related to the presence of the respective antigens.

### Immunoassays

Both the polypeptides described herein and the antibodies that bind with specificity to the polypeptides are useful as reagents, as capture agents or labeling agents, in assays to detect a target peptide or antibody. In general, the target molecule can be quantified by a variety of immunoassay methods. Moreover, the immunoassays can be performed in any of several configurations.

Immunoassays often utilize a labeling agent to specifically bind to and label the binding complex formed by the capture agent and the analyte. The labeling agent may itself be one of the moieties comprising the antibody/ analyte complex. Thus, the labeling agent may be a labeled peptide or a labeled anti-peptide antibody. Alternatively, the labeling agent may be a third moiety, such as another antibody, that specifically binds to the antibody/peptide complex, or to a modified capture group (*e.g.,* biotin) which is covalently linked to the peptide or anti-peptide antibody.

Alternatively, the labelling agent can be a streptavidin molecule which has a fluorescent dye on it and onto which are captured the peptides complexed with MHC (HLA) molecules. These reagents can be used to count single T cells specific for the peptides using commonly used equipment such as flow cytometers, thus providing precise quantitation and phenotype information on the immune response as described by Altman, J.D. *et al., Science* 274(5284):94-96 (1996).

In a preferred embodiment, the labeling agent is an antibody that specifically binds to the capture agent. Such agents are well known to those of skill in the art, and most typically comprise labeled antibodies that specifically bind antibodies of the particular animal species from which the capture agent is derived, such as an anti-idiotypic antibody, or antibodies against a peptide when the peptide is the capture agent. Thus, for example, where the capture agent is a mouse derived anti-peptide antibody, the label agent may be a goat anti-mouse IgG, *i.e.,* an antibody specific to the constant region of the mouse antibody.

Other proteins capable of specifically binding immunoglobulin constant regions, such as streptococcal protein A or protein G are also used as the labeling agent. These proteins are normal constituents of the cell walls of streptococcal bacteria. They exhibit a strong non immunogenic reactivity with immunoglobulin constant regions from a variety of species.

Throughout the assays, incubation and/or washing steps may be required after each combination of reagents. Incubation steps can vary from about five seconds to several hours, preferably from about five minutes to about 24 hours. However, the incubation time will depend upon the assay format, analyte, volume of solution, concentrations, and the like. Usually, the assays are carried out at ambient temperature, although they can be conducted over a range of temperatures, such as 5 C to 45 C.

### Non Competitive Assay Formats

Immunoassays for detecting a peptide or an antibody to a peptide may be either competitive or noncompetitive. Noncompetitive immunoassays are assays in which the amount of captured analyte (*e.g.,* anti-peptide antibody) is directly measured. In one preferred "sandwich" assay, for example, the capture agent (*e.g.,* immunogenic peptide antibodies) is bound directly to a solid substrate where they are immobilized. These immobilized peptides capture antibodies present in a test sample, such as biological fluid, most preferably blood serum. The antibody thus immobilized is then bound by a labeling agent, such as a second antibody bearing a label. Alternatively, the second antibody may lack a label, but it may, in turn, be bound by a labeled third antibody specific to antibodies of the species from which the second antibody is derived.

Sandwich assays for a peptide or antibody can also be constructed. As described above, the immobilized peptide specifically binds to the antibody present in the sample. A labeled antibody then binds to the already bound antibody. Free labeled antibody is washed away and the remaining bound labeled antibody is detected (*e.g*., using a gamma detector where the label is radioactive).

### Competitive Assay Formats

In competitive assays, the amount of analyte (*e.g*., immunogenic peptide or antibody to an immunogenic peptide) present in the sample is measured indirectly by measuring the amount of an added (exogenous) analyte displaced (or competed away) from a capture agent (*e.g.,* an antibody or peptide) by the analyte present in the sample. In one competitive assay, a known amount of analyte is added to the sample and the sample is contacted with a capture agent, such as a peptide that specifically binds the analyte. The amount of analyte bound to the peptide is inversely proportional to the concentration of analyte present in the sample.

In a preferred embodiment, the capture agent is immobilized on a solid substrate. The amount of analyte bound to the capture agent is determined either by measuring the amount of antibody present in an antibody/peptide complex or, alternatively, by measuring the amount of remaining uncomplexed antibody. The amount of peptide in a sample to be assayed can also be detected by providing exogenous labeled peptide to the assay.

A hapten inhibition assay is another preferred competitive assay. In this assay, a known analyte, in this case one or more of the peptides described herein, is immobilized on a solid substrate. A known amount of anti-peptide antibody is added to the sample, and the sample is then contacted with the immobilized peptide. In this case, the amount of antibody bound to the immobilized polypeptide is proportional to the amount of peptide present in the sample. Again, the amount of immobilized antibody is detected by quantitating either the immobilized fraction of antibody or the fraction of the antibody that remains in solution. Detection may be direct where the antibody is labeled, or indirect where a labeled moiety is subsequently added which specifically binds to the antibody as described above. One of skill will appreciate that the role of the peptide and antibody can be reversed to achieve the same effect for the quantitation of the antibody.

One or more of the polypeptides described herein or, alternatively, one or more of the antibodies to the polypeptides is preferably quantified in a biological sample, such as a biological fluid or tissue sample derived from a patient. The detection of the peptides or antibodies indicates that the individual from whom the biological sample was taken is mounting an immune response to the virus. A determination of the quantity of antibodies or protein present in the biological sample provides an indication of the degree of immunity or response to treatment and can therefore be used as a prognostic evaluation.

The sample to be tested or analyzed may be obtained from any biological source and is preferably taken from a human or animal capable of being infected with or harboring the hepatitis A virus. For example, the sample may be a cell sample, tissue sample or biological fluid, such as whole blood, blood serum, blood plasma, urine, semen, saliva, sputum, cerebrospinal fluid, lacrimal fluid, fermentation fluid, lymph fluid, tissue culture fluid, ascites fluid, synovial fluid, pleural fluid, and the like. The preferred biological sample is a biological fluid from which cells can be removed. The most preferred samples are blood plasma or serum. The biological sample may also be a laboratory research sample such as a cell culture supernatant, viral isolate or viral concentrate. The sample is collected or obtained using methods well known to those skilled in the art.

The sample may be diluted, purified, concentrated, filtered, dissolved, suspended, or otherwise manipulated prior to use in the assay. Preferably, a sample containing particulate matter is diluted, filtered, or both diluted and filtered prior to use. The preferred diluent is a buffer solution. Any of a number of standard aqueous buffer solutions, employing one of a variety of buffers, such as phosphate, TRIS detergent, or the like, at physiological pH can be used.

The sample size for the biological fluid sample is preferably between approximately 0.5 µl and 1 ml. A preferred biological fluid sample size is between approximately 1 and 100 µl. Most preferably, the volume of the biological fluid sample is approximately 10 to 50 µl.

After reactivity with one or more of the reagents described herein, the target peptide or antibody in the sample can be detected and quantified by any of a number of means well known to those of skill in the art. These include analytic biochemical methods such as spectrophotometry, radiography, electrophoresis, capillary electrophoresis, high performance liquid chromatography (HPLC), thin layer chromatography (TLC), hyperdiffusion chromatography, and the like, and various immunological methods such as fluid or gel precipitation reactions, immunodiffusion (single or double), immunoelectrophoresis, radioimmunoassays (RIAs), enzyme-linked immunosorbent assays (ELISAs), immunofluorescent assays, and the like.

### Other Assay Formats

Western blot analysis can also be used to detect and quantify the presence of target peptide in the sample. The technique generally includes separating sample products by gel electrophoresis on the basis of molecular weight, transferring the separated proteins to a suitable solid support (such as a nitrocellulose filter, a nylon filter, or derivatized nylon filter), and incubating the sample with the antibodies that specifically bind the peptides. The anti-peptide antibodies specifically bind to a peptide fixed on the solid support. These antibodies are directly labeled or, alternatively, they may be subsequently detected using labeled antibodies (*e.g*., labeled sheep anti-mouse antibodies where the antibody to a peptide is a murine antibody) that specifically bind to the anti-peptide antibody.

Other assay formats include liposome immunoassays (LIAs), which use liposomes designed to bind specific molecules (*e.g.,* antibodies) and release encapsulated reagents or markers. The released chemicals are then detected according to standard techniques.

### Labels

The labeling agent used to label the polypeptide or antibody can be, *e.g.,* a peptide, a monoclonal antibody, a polyclonal antibody, an immunogenic peptide or a mosaic polypeptide of immunogenic peptides, or complex such as those described herein, or a polymer such as an affinity matrix, carbohydrate or lipid. Detection may proceed by any known method, such as immunoblotting, western analysis, gel-mobility shift assays, fluorescent *in situ* hybridization analysis (FISH), tracking of radioactive or bioluminescent markers, nuclear magnetic resonance, electron paramagnetic resonance, stopped-flow spectroscopy, column chromatography, capillary electrophoresis, or other methods which track a molecule based upon an alteration in size and/or charge. The particular label or detectable group used in the assay is not a critical aspect of the invention The detectable group can be any material having a detectable physical or chemical property. Such detectable labels have been well-developed in the field of immunoassays and, in general, any label useful in such methods can be applied to the present invention. Thus, a label is any composition detectable by spectroscopic, photochemical, biochemical, immunochemical, electrical, optical or chemical means. Useful labels in the present invention include magnetic beads (*e.g.* Dynabeads™), fluorescent dyes (*e.g.,* fluorescein isothiocyanate, Texas red, rhodamine, and the like), radiolabels (*e.g.,* ³H, ¹²⁵I, ³⁵S, ¹⁴C, or ³²P), enzymes (*e.g,* LacZ, CAT, horse radish peroxidase, alkaline phosphatase and others, commonly used as detectable enzymes, either in an EIA or in an ELISA), and colorimetric labels such as colloidal gold or colored glass or plastic (*e.g.* polystyrene, polypropylene, latex, *etc*.) beads. The label may be coupled directly or indirectly to the desired component of the assay according to methods well known in the art. As indicated above, a wide variety of labels may be used, with the choice of label depending on the sensitivity required, ease of conjugation of the compound, stability requirements, available instrumentation, and disposal provisions.

Non-radioactive labels are often attached by indirect means. Generally, a ligand molecule, such as biotin, is covalently bound to the molecule. The ligand then binds to an anti-ligand, such as streptavidin, molecule which is either inherently detectable or covalently bound to a signal system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labeled, naturally occurring anti-ligands. Alternatively, any haptenic or antigenic compound can be used in combination with an antibody.

The molecules can also be conjugated directly to signal generating compounds, *e.g.*, by conjugation with an enzyme or fluorophore. Enzymes of interest as labels will primarily be hydrolases, particularly phosphatases, esterases and glycosidases, or oxidoreductases, particularly peroxidases. Fluorescent compounds include fluorescein and its derivatives, rhodamine and its derivatives, dansyl, umbelliferone, etc. Chemiluminescent compounds include luciferin, and 2,3-dihydrophthalazinediones, e.g., luminol. For a review of various labelling or signal producing systems which may be used, see, U.S. Patent No. 4,391,904.

Means of detecting labels are well known to those of skill in the art. Thus, for example, where the label is a radioactive label, means for detection include a scintillation counter or photographic film as in autoradiography. Where the label is a fluorescent label, it may be detected by exciting the fluorochrome with the appropriate wavelength of light and detecting the resulting fluorescence, *e.g.,* by microscopy, visual inspection, via photographic film, by the use of electronic detectors such as charge coupled devices (CCDs) or photomultipliers and the like. Similarly, enzymatic labels are detected by providing appropriate substrates for the enzyme and detecting the resulting reaction product. Finally, simple colorimetric labels may be detected simply by observing the color associated with the label. Thus, in various dipstick assays, conjugated gold often appears pink, while various conjugated beads appear the color of the bead.

Some assay formats do not require the use of labeled components. For instance, agglutination assays can be used to detect the presence of the target antibodies. In this case, antigen-coated particles are agglutinated by samples comprising the target antibodies. In this format, none of the components need be labeled and the presence of the target antibody is detected by simple visual inspection.

### Solid Phase

As mentioned above, depending upon the assay, various components, including the immunogenic polypeptide, anti-peptide antibody, or anti-idiotypic antibody, may be bound to a solid surface. Many methods for immobilizing biomolecules to a variety of solid surfaces are known in the art. For instance, the solid surface may be a membrane (*e.g*., nitrocellulose), a microtiter dish (*e.g*., PVC, polypropylene, or polystyrene), a test tube (glass or plastic), a dipstick (*e.g*. glass, PVC, polypropylene, polystyrene, latex, and the like), a microcentrifuge tube, or a glass, silica, plastic, metallic or polymer bead. The desired component may be covalently bound, or noncovalently attached through nonspecific bonding.

A wide variety of organic and inorganic polymers, both natural and synthetic may be employed as the material for the solid surface. Illustrative polymers include polyethylene, polypropylene, poly(4-methylbutene), polystyrene, polymethacrylate, poly(ethylene terephthalate), rayon, nylon, poly(vinyl butyrate); polyvinylidene difluoride (PVDF), silicones, polyformaldehyde, cellulose, cellulose acetate, nitrocellulose, and the like. Other materials which may be employed, include paper, glass, ceramics, metals, metalloids, semiconductive materials, cements or the like. In addition, substances that form gels, such as proteins (*e.g.,* gelatins), lipopolysaccharides, silicates, agarose, and polyacrylamides can be used. Polymers which form several aqueous phases, such as dextrans, polyalkylene glycols or surfactants, such as phospholipids, long chain (12 to 24 carbon atoms) alkyl ammonium salts and the like are also suitable. Where the solid surface is porous, various pore sizes may be employed depending upon the nature of the system.

In preparing the surface, a plurality of different materials may be employed, *e.g.,* as laminates, to obtain various properties. For example, protein coatings, such as gelatin can be used to avoid non specific binding, simplify covalent conjugation, enhance signal detection or the like.

If covalent bonding between a compound and the surface is desired, the surface will usually be polyfunctional or be capable of being polyfunctionalized. Functional groups which may be present on the surface and used for linking can include carboxylic acids, aldehydes, amino groups, cyano groups, ethylenic groups, hydroxyl groups, mercapto groups and the like. The manner of linking a wide variety of compounds to various surfaces is well known and is amply illustrated in the literature.

### Pharmaceutical Compositions

Vaccine and other pharmaceutical compositions containing one or more of the polypeptides or antibodies described herein in a pharmaceutically acceptable carrier are provided. The compositions are useful in therapeutic and prophylactic methods for the treatment, prevention, or reduction of HEV infection in humans. Such compositions are suitable for use in a variety of drug delivery systems. Suitable formulations are found in *Remington's Pharmaceutical Sciences,* Mack Publishing Company, Philadelphia, PA, 17th ed. (1985). A brief review of methods for drug delivery is provided by Langer, *Science* 249:1527-1533 (1990).

The compositions are suitable for single administrations or a series of administrations. When given as a series, inoculations subsequent to the initial administration are given to boost the immune response and are typically referred to as booster inoculations.

The pharmaceutical compositions are intended for parenteral, topical, oral or local administration. Preferably, the pharmaceutical compositions are administered parenterally, *e.g*., intravenously, subcutaneously, intradermally, or intramuscularly. Thus, the invention provides compositions for parenteral administration that comprise a solution of the agents described above dissolved or suspended in an acceptable carrier, preferably an aqueous carrier. A variety of aqueous carriers may be used, *e.g.,* water, buffered water, 0.4% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10% to 95% of active ingredient and more preferably at a concentration of 25% to 75 % of active ingredient.

For aerosol administration, the polypeptides are preferably supplied in finely divided form along with a surfactant and propellant. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, such as the inclusion of lecithin for intranasal delivery.

The amount administered to the patient will vary depending upon what is being administered, the state of the patient and the manner of administration. In therapeutic applications, compositions are administered to a patient already infected with the HEV virus in an amount sufficient to inhibit spread of the virus, or at least partially arrest the symptoms of the disease and its complications. An amount adequate to accomplish this is defined as "therapeutically effective dose." Amounts effective for this use will depend on the severity of the disease, the particular composition, and the weight and general state of the patient. Generally, the dose will be in the range of about 100 µg to about 3000 µg per day, preferably about 1500 µg per day, for a 70 kg patient.

More preferably, the polypeptide is used prophylactically as a vaccine. All of the immunogenic polypeptides disclosed herein can be used as vaccines, either alone, in combination or in combination, as in a multiepitope or polyepitope vaccine. The immune response may include the generation of antibodies, activation of cytotoxic T lymphocytes (CTL) against cells presenting the immunogenic polypeptides, or another mechanism well known in the art. Preferably, the immune response includes the generation of neutralizing antibodies. The preferred dose will be in the range of 100 µg to about 3000 µg per day, preferably about 1500 µg per day, administered in one to six doses.

In a preferred embodiment, the immunogenic polypeptides are covalently attached (conjugated) to a carrier protein as described above. Useful carrier proteins include, but are not limited to, thyroglobulin, albumins such as human serum albumin, tetanus toxoid, polyamino acids such as poly(D-lysine:D-glutamic acid), influenza, hepatitis B virus core protein, hepatitis B virus recombinant vaccine. The vaccines can also contain a physiologically tolerable (acceptable) diluent such as water, phosphate buffered saline, or saline, and further typically include an adjuvant. Adjuvants such as incomplete Freund's adjuvant, aluminum phosphate, aluminum hydroxide, or alum are materials well known in the art.

### DNA Vaccines

In addition, DNA or RNA encoding the immunogenic polypeptides or the antibodies of the present invention may be introduced into patients to obtain an immune response to the immunogenic polypeptides which the nucleic acid encodes. *See,* Wolff, *et al., Science* 247: 1465-1468 (1990) which describes the use of nucleic acids to produce expression of the immunogenic polypeptides which the nucleic acids encode, the teachings of which are incorporated herein by reference. Vaccines composed of DNA or RNA encoding immunogenic polypeptides are commonly referred to in the art as DNA vaccines.

Vaccine compositions containing the immunogenic polypeptides and nucleic acids of the invention are administered to a patient to elicit a protective immune response against the polypeptide. A "protective immune response" is one which prevents or inhibits the spread of HEV and, thus, at least partially prevents the symptoms of the disease and its complications. An amount sufficient to accomplish this is defined as an "immunogenically effective dose." Amounts effective for this use will depend on the composition, the manner of administration, the weight and general state of health of the patient, and the judgment of the prescribing physician. For peptide compositions, the general range for the initial immunization (that is for therapeutic or prophylactic administration) is from about 100 µg to about 3000 µg per day, preferably about 1500 µg per day, followed by boosting dosages of the peptide pursuant to a boosting regimen over weeks to months depending upon the patient's response and condition, *e.g.,* by measuring levels of HEV in the patient's blood. For nucleic acids, the same range of doses is preferred.
The invention is further illustrated by the following examples, which are not to be construed in any way as imposing limitations upon the scope thereof.

The invention thus covers the use of HEV polypeptides as defined in claim 1 as well as the specific HEV polypeptides per se, as defined in Claim 8.

### Examples

### Methods and Materials

### Cell culture

PLC/PRF/5, a human hepatocarcinoma cell line, was grown in Dulbecco's modified Eagle medium (Gibco BRL, Grand Island, NY) supplemented with 10% heat-inactivated fetal bovine serum (HyClone, Laboratory, Int., Logan, Utah), and incubated at 37°C with 5% CO₂. For the *in vitro* neutralization assay, described below, trypsinized cells were seeded into 24-well, flat-bottom culture plates at a concentration of 10⁵ cells per well and incubated overnight to form cell monolayers.

### Virus stocks

The inocula of the HEV Burma, Pakistan, Morocco, and Mexico strains are described in Meng et al., 1998. The HEV US strain was obtained from a fecal sample collected from a 62-year-old white male suffering from acute viral hepatitis who had not recently traveled outside the United States (Kwo et al., 1997; Schlauder et al., 1998). This inoculum was prepared as described in Meng et al., 1998.

### Synthetic peptides

Fifty one overlapping 30-mer peptides (P1-P51) encompassing the pORF2 protein between amino acids 221 and 660 (SEQ. ID NO.:1) were synthesized by FMOC chemistry on an ACT Model MPS 350 multiple peptide synthesizer (Advanced Chemtech, Louisville, KY) according to the manufacturer's protocols. The synthetic peptides were characterized by amino acid analysis, high performance liquid chromatography, and capillary electrophoresis. For animal immunization, each of the peptides was conjugated with a carrier protein, bovine serum albumin (BSA), by 1-ethyl-3-(3-dimethylaminopropyl) carbodiimide hydrochloride (EDC) coupling methods using a commercially available kit (PIERCE, Rockford, IL).

### Construction of HEV recombinant plasmids

Thirty-one HEV recombinant plasmids were constructed with pGEX-4T-2 vector (Pharmacia Biotech Inc., Piscataway, NJ) and different sizes of PCR fragments which were amplified by PCR walking technique from an HEV Burma plasmid containing the whole ORF2 sequence. The primers were selected based on HEV Burma sequence (Tam et al., 1990) and modified to contain BamH I or Xho I restriction sites to facilitate cloning.

Amplification was performed with the Expand™ High Fidelity PCR System (Boehringer Mannheim, GmbH, Germany). PCR products were purified with QIAquick PCR Purification Kit (QIAGEN Inc., Valencia, CA). Both purified PCR products and pGEX-4T-2 vector were digested with BamH I and Xho I (Boehringer Mannheim) in buffer B at 37°C overnight, ligated with T4 DNA ligase (Pharmacia Biotech) at 16°C overnight, and then used to transform *E. coli* competent JM109 cells (Promega, Madison, WI). After cloning, recombinant plasmids were recovered from transformants by using the Wizard Miniprep DNA Purification system (Promega). The presence of an insert was confirmed by PCR using two primers derived from regions flanking the multiple cloning site of pGEX-4T-2. The primary structure of the inserts was finally confirmed by DNA sequencing with an automated 373 or 377 DNA sequencer (ABI, Foster City, CA).

### Production of HEV-GST fusion proteins

*E. coli* JM109 cells transformed with the recombinant plasmids were grown at 37°C overnight in Luria broth (LB) medium containing 50 µg/ml ampicillin. The overnight culture was diluted 20 times with fresh LB medium containing the same concentration of ampicillin and grown at 37°C for 3 to 4 hours until an optical density (OD) value of 0.6 to 1.0 at 600 nm was reached. The gene expression was induced by adding isopropyl-(-D-thiogalactopyronoside) (IPTG, Sigma Chemical Co., St Louis, MO) into the culture to a final concentration of 1 mM. After 4 hours of incubation at 37°C with constant shaking, the cells were pelleted by centrifugation at 6000g for 15 minutes at 4°C and then resuspended with 3 ml of lysis buffer (50 mM Tris pH 8.0, 1 mM EDTA,100 mM NaCl) for each gram of packed cells. The suspension was incubated in ice for 30 minutes with a final concentration of 0.2 mM phenylmethylsulfonyl fluoride (PMSF, Sigma) and 0.5 mg/ml of lysozyme (Sigma). Then, 4 mg of deoxycholic acid were added per gram of *E*. *coli* cells while stirring continuously in room temperature for 5 minutes. The lysate was incubated with 20 U/ml of DNAse (Boehringer Mannheim) in room temperature until it was no longer viscous and centrifuged at 10,000g for 20 minutes at 4°C. The supernatant was transferred to a fresh tube and purified with Bulk and Redipack GST Purification Modules (Pharmacia Biotech). The pellet containing insoluble HEV-GST fusion protein was washed completely, resuspended and homogenized with phosphate-buffered saline (PBS), and stored in aliquots at -70°C. Meanwhile, GST protein was prepared from pGEX-4T-2 vector as control.

### Western Immunoblot analysis for HEV recombinant proteins

Aliquots of each homogenized HEV-GST fusion proteins were separated by electrophoresis on precast 12% sodium dodecyl sulfate (SDS)-polyacrylamide gels (Bio-Rad, Richmond, CA) followed by blotting onto nitrocellulose membranes (Bio-Rad). The nitrocellulose membranes were incubated overnight with blocking buffer containing 10% normal goat serum, 1% bovine serum albumin (BSA) and 0.05% Tween 20 in 0.01M PBS, and then incubated for 1 hour with serum samples collected from a cynomalgus macaque experimentally-infected with the HEV Pakistan strain (SAR-55), diluted 1:100 in blocking buffer. The membranes were rinsed three times with wash buffer (PBS with 0.05% Tween 20), and incubated for 1 hour with affinity-purified goat anti-human immunoglobulin G (IgG, Pierce) conjugated with horseradish peroxidase, diluted 1:6000 in blocking buffer. After three times of washing as above, color development was carried out with 3,3'-diaminobenzidine as a substrate (Bio-Rad).

### Immunization of mice with HEV synthetic peptides and recombinant polypeptides

Each of the BSA-conjugated peptides and the HEV-GST fusion proteins was emulsified with an adjuvant, TiterMax (CytRx, Atlanta, GA), in equal volume, and then used to immunize a group of 3-4 female Hsd NIHS mice of 6-9 weeks old. The mice were inoculated subcutaneously at two sites on the back with total of 100 µl of the emulsion containing 50 µg of the conjugated peptide or the fusion protein. Four weeks later, the mice were boosted with an intraperitoneal injection of 10 µg of the same peptide or protein diluted in 100 µl of PBS. After 7 days of the booster injection, the mice were bled from the heart. The immune serum samples obtained from each group of mice were pooled together and inactivated by heating at 60°C for 30 minutes. Aliquots were prepared and stored at -70°C for further test. Immune serum samples against BSA and GST were prepared with the same procedure.

### Enzyme immunoassays for anti-HEV antibodies

The protocol used for detecting antibodies against HEV synthetic peptides is described in Khudyakov et al., 1999. Generally, synthetic peptides (110 µl) at a concentration of 5 µg/ml in 0.1 M phosphate-buffered saline (PBS), pH 7.5, were adsorbed to microtiter wells (Immulon II; Dynatech Laboratories, Inc.) at room temperature for 12 hours. Serum was diluted 1:100 in PBS containing 0.1% Tween 20 and 10% normal goat serum (PBS-T). One hundred microliters of diluted serum was added to each well and incubated for 1 hour at 37 C. The binding of antibodies to the peptides was identified with affinity-purified antibodies to human IgM coupled to horseradish peroxidase (Boehringer Mannheim, Indianapolis, Ind.) by adding 100µl of a 1:10,000 or 1:5,000 dilution, respectively, in PBS-T and incubating for 1 hour at 37 C. The cutoff, expressed as a PIN ratio and equal to 3.0, was statistically established individually for each peptide as the mean of the result with negative controls plus at least 3.5 standard deviations above the mean, where P represents the optical density at 493 nm (OD₄₉₃) of anti-HEV-positive specimens and N represents the OD of negative controls. Each serum specimen in every experiment was also tested with an irrelevant peptide (no. 1546) with the sequent PMSMDTSDETSEGATFLSLS derived from a small ORF within the hepatitis G virus minus-sense RNA. As an additional criterion, the ratio between the OD₄₉₃ for each HEV peptide and the OD₄₉₃ for this irrelevant peptide found for each serum specimen was used. HEV peptides were considered specifically immunoreactive with serum specimens when this ratio was greater than 2

### In vitro PCR-based seroneutralization assay

This assay is described in Meng et al.,1997;1998. Briefly, approximately 100 cell culture infectious doses of an HEV inoculum diluted in 100 µl of Hanks' solution were mixed with 100 µl of an immune serum sample at a dilution of 1:10. After incubation at 37°C for 1 hour, the mixture was inoculated onto a cell monolayer of PLC/PRF/5. After adsorption for 2 hours at 37°C, the cells were washed three times with Hanks' solution followed by immediate RNA extraction with TRIzol reagent (Gibco BRL) according to the manufacturer's instructions. Reverse transcription, nested PCR was performed by using a set of universal HEV PCR primers. The outer primers were YK-1291 (5'- GTT GTC TCA GCC AAT GGC GAG CC) (SEQ. ID NO.: 2) and YK-1294 (5'- GCC TGC GCG CCG GTC GCA ACA) (SEQ. ID NO.: 3). The internal primers were YK-1292 (5'- TGG AGA ATG CTC AGC AGG ATA A) (SEQ. ID NO.: 4) and YK-1293 (5'- TAA GTG GAC TGG TCG TAC TCG GC) (SEQ. ID NO.: 5). Both the first-round and second-round amplifications were carried out according to the following cycling program: denaturation at 94°C for 45 seconds, annealing at 60°C for 20 seconds, extension at 72°C for 60 seconds, for 30 cycles. Amplicons were separated by agarose gel electrophoresis with size markers and visualized by ethidium bromide fluorescence. Neutralization was determined by the absence of detectable HEV RNA in the inoculated cell culture. A normal mouse serum control, anti-BSA or anti-GST serum control, virus control, and uninoculated cell control were processed for detection of HEV RNA at the same time.

### Results

HEV neutralizing antigenic epitope(s) could not be modeled with the synthetic peptides The 51 immune serum samples against BSA-conjugated HEV synthetic peptides (P1-P51) were tested by both ELISA and the *in vitro* neutralization assay. As shown in Table 1, all the serum samples were immunoreactive to the carrier protein BSA, indicating that the mice developed immune responses to the antigens. However, only 30 of 51 (59%) serum samples contained detectable antibodies to their respective synthetic peptides. These reactive samples appeared to cluster into 5 groups: group 1 with anti-P1 to anti-P4, group 2 with anti-P11 to anti-P16, group 3 with anti-P19 to anti-P28, group 4 with anti-P33 to anti-P43, and group 5 with only anti-P51. Nevertheless, when the immune serum samples were tested by the *in vitro* neutralization assay, none of them demonstrated neutralizing activity to both the HEV Burma and Mexico strains. Since pooled antibodies, sometimes, can increase antibody functions, the immune serum samples in the groups 1, 2, 3, and 4 were pooled together respectively, and tested again by the *in vitro* neutralization assay. No neutralizing activity was detected. Therefore, antibodies obtained by immunization of mice with HEV synthetic peptides could be detected by ELISA but not by the *in vitro* neutralization assay. These data implied that the HEV neutralizing antigenic epitope(s) might be distinct from the epitopes which can induce ELISA-detectable antibodies.

**Table 1. Detection of antibodies against BSA-conjugated HEV synthetic peptides by ELISA and neutralization assay**

| Peptide | Location | ELISA with antigen | | Neutralization assay with HEV strain | |
|---|---|---|---|---|---|
| | (amino acids) | BSA | Peptide | Burma | Mexico |
| P1 | 221-250 | + | + | - | - |
| P2 | 232-262 | + | + | - | - |
| P3 | 237-266 | + | + | - | - |
| P4 | 242-271 | + | + | - | - |
| P5 | 245-274 | + | - | - | - |
| P6 | 254-283 | + | - | - | - |
| P7 | 262-291 | + | - | - | - |
| P8 | 272-300 | + | - | - | - |
| P9 | 282-311 | + | - | - | - |
| P10 | 291-320 | + | - | - | - |
| PI1 | 301-330 | + | + | - | - |
| P12 | 309-338 | + | + | - | - |
| P13 | 314-343 | + | + | - | - |
| P14 | 318-347 | + | + | - | - |
| P15 | 326-355 | + | + | - | - |
| P16 | 334-363 | + | + | - | - |
| P17 | 344-373 | + | - | - | - |
| P18 | 353-382 | + | - | - | - |
| P19 | 381-410 | + | + | - | - |
| P20 | 391-420 | + | + | - | - |
| P21 | 398-427 | + | + | - | - |
| P22 | 403-432 | + | + | - | - |
| P23 | 408-437 | + | + | - | - |
| P24 | 415-444 | + | + | - | - |
| P25 | 421-450 | + | + | - | - |
| P26 | 431-460 | + | + | - | - |
| P27 | 438-467 | + | + | - | - |
| P28 | 442-471 | + | + | - | - |
| P29 | 450-479 | + | - | - | - |
| P30 | 457-486 | + | - | - | - |
| P31 | 466-495 | + | - | - | - |
| P32 | 475-504 | + | - | - | - |
| P33 | 483-512 | + | + | - | - |
| P34 | 491-520 | + | + | - | - |
| P35 | 500-529 | + | + | - | - |
| P36 | 513-542 | + | - | - | - |
| P37 | 521-550 | + | + | - | - |
| P38 | 528-557 | + | + | - | - |
| P39 | 538-567 | + | + | - | - |
| P40 | 543-572 | + | + | - | - |
| P41 | 551-580 | + | + | - | - |
| P42 | 562-591 | + | + | - | - |
| P43 | 573-602 | + | + | - | - |
| P44 | 584-613 | + | - | - | - |
| P45 | 592-621 | + | - | - | - |
| P46 | 601-630 | + | - | - | - |
| P47 | 610-639 | + | - | - | - |
| P48 | 615-644 | + | - | - | - |
| P49 | 621-650 | + | - | - | - |
| P50 | 626-655 | + | - | - | - |
| P51 | 631-660 | + | + | - | - |

### Expression of HEV-GST fusion proteins

Thirty one fragments encompassing different regions of the whole HEV Burma ORF2 sequence were separately amplified by PCR, and then cloned into the prokaryotic expression vector pGEX-4T-2. The inserted fragment in each clone was confirmed by DNA sequencing. After induction with IPTG, abundant amounts of the HEV-GST fusion proteins were expressed, as evidenced by the absence from *E. coli* JM109 transformed with pGEX-4T-2 vector itself. The estimated molecular weights of the expressed fusion proteins were consistent with the predicted sizes, including the 26 kDa of GST (Table 2).

**Table 2. Recombinant HEV-GST fusion proteins and their Western blot reactivity to the immune serum sample**

| HEV-GST fusion protein | Location (amino acids) | Number of amino acids | Predicted size (kDa) | Western Blot (immune serum) |
|---|---|---|---|---|
| pA1 | 1-103 | 103 | 37.3 | + |
| pA2 | 42-150 | 109 | 38.0 | + |
| pA3 | 72-174 | 103 | 37.3 | + |
| pA4 | 113-236 | 124 | 39.6 | + |
| pA5 | 153-265 | 113 | 38.4 | - |
| pA6 | 189-305 | 117 | 38.9 | - |
| pA7 | 245-356 | 112 | 38.3 | - |
| pA8 | 274-384 | 111 | 38.1 | - |
| pA9 | 336-444 | 109 | 38.0 | + |
| pA10 | 363-475 | 112 | 38.3 | + |
| pA11 | 393-507 | 115 | 38.7 | + |
| pA12 | 421-540 | 120 | 39.3 | + |
| pA13 | 452-580 | 129 | 40.2 | - |
| pA14 | 499-617 | 119 | 39.1 | - |
| pA15 | 541-660 | 120 | 39.2 | + |
| pF1 | 1-417 | 417 | 71.9 | + |
| pF2 | 113-507 | 395 | 69.5 | + |
| pF3 | 189-580 | 392 | 69.1 | + |
| pF4 | 274-660 | 387 | 68.6 | + |
| pN309 | 309-660 | 352 | 64.7 | + |
| pN336 | 336-660 | 325 | 61.8 | + |
| pN364 | 364-660 | 297 | 58.7 | + |
| pN393 | 393-660 | 268 | 55.5 | + |
| pN421 | 421-660 | 240 | 52.4 | + |
| pN452 | 452-660 | 209 | 49.0 | + |
| pN499 | 499-660 | 162 | 43.8 | + |
| pC617 | 274-617 | 344 | 63.8 | + |
| pC580 | 274-580 | 307 | 59.8 | + |
| pC540 | 274-540 | 267 | 55.4 | + |
| pC507 | 274-507 | 234 | 51.7 | + |
| pB166 | 452-617 | 166 | 44.3 | + |

Most of the proteins were produced as inclusion bodies. Some of them, such as pA1, pA2, pA3, pA4, pA11, pA12, pA13, pA14, pA15, pF2, pN309, pN393, pN421, pN452, and pB166 were partially soluble. All of the partially soluble proteins located at the N- or C-terminal part of the HEV pORF2 (except pF2) had a molecular weight of no more than 55.5 kDa in size including the GST portion (except pF2 and pN309). The antigenic reactivity of the recombinant proteins was analysed by Western immunoblot assay with serum samples obtained from an experimentally infected cynomolgus macaque inoculated with HEV Pakistan strain SAR-55. No proteins were recognized by the serum sample collected before inoculation, while 25 out of the 31 proteins were reactive to the serum sample collected at day 54 after inoculation (Table 2). The data ascertained the specificity of the recombinant fusion proteins with HEV characteristics. Therefore, the proteins were used to immunize mice to generate immune serum samples for neutralization assay.

### HEV neutralizing antigenic epitope (s) is located at the C-terminal part of pORF2

Fifteen immune serum samples against the about 100 aa long recombinant proteins (pA1-pA15) and 4 immune serum samples against the about 400 aa long recombinant proteins (pF1-pF4) were first tested by ELISA with a purified GST antigen. As shown in Table 3, all the serum samples were immunoreactive to GST, indicating that the immunization of mice was successful with these recombinant proteins of GST fusion properties. Then, the *in vitro* neutralization assay was performed with both the HEV Burma and Mexico strains to determine the neutralizing activity. Similar to the synthetic peptides, none of the about 100 aa long proteins elicited neutralizing antibodies. For the immune serum samples against the four about 400 aa long proteins, 3 of them respectively against pF1 (aa 1-417), pF2 (aa 113-507), and pF3 (aa 189-580) also failed to present neutralizing activity. Only the serum sample against pF4, which is located at the extreme C-terminal part (aa 274-660) of pORF2, neutralized both Burma and Mexico strains in the *in vitro* neutralization assay. However, 3 groups of pooled immune serum samples against pA8-pA15, pF2 and pA12-pA15, pF3 and pA14-pA15, whose corresponding sequences covered aa 274-660 respectively, did not show any neutralizing activity as well (Table 3). These data show that the HEV neutralizing antigenic epitope(s) are located at the C-terminal part of pORF2, and also evidence that this epitope(s) is highly conformation dependent.

**Table 3. Neutralizing activity of the immune serum samples against about 100 and about 400 amino acid long HEV recombinant proteins**

| Anti-HEV | ELISA | Neutralization assay with | |
|---|---|---|---|
| recombinant protein | with GST | HEV strain | |
| | | Burma | Mexico |
| Anti-pA1 | + | - | - |
| Anti-pA2 | + | - | - |
| Anti-pA3 | + | - | - |
| Anti-pA4 | + | - | - |
| Anti-pA5 | + | - | - |
| Anti-pA6 | + | - | - |
| Anti-pA7 | + | - | - |
| Anti-pA8 | + | - | - |
| Anti-pA9 | + | - | - |
| Anti-pA10 | + | - | - |
| Anti-pA11 | + | - | - |
| Anti-pA12 | + | - | - |
| Anti-pA13 | + | - | - |
| Anti-pA14 | + | - | - |
| Anti-pA15 | + | - | - |
| Anti-pF1 | + | - | - |
| Anti-pF2 | + | - | - |
| Anti-pF3 | + | - | - |
| Anti-pF4 | + | + | + |
| Pooled anti-pA8, 9, 10, 11, 12, 13, 14, 15 | + | - | - |
| Pooled anti-pF2, anti-pA12, 13, 14, 15 | + | - | - |
| Pooled anti-F3, anti-pA14, 15 | + | - | - |

### HEV neutralizing antigenic epitope(s) was mapped within the sequence of aa 452-617 of pORF2

For fine mapping of the HEV neutralizing antigenic epitope(s), an additional 12 immune serum samples against recombinant proteins truncated from pF4 in N-, or C-, or both N and C-terminus were tested by ELISA and the *in vitro* neutralization assay. The results are shown in Table 4.

**Table 4. Neutralizing activity of the immune serum samples against the truncated pF4 recombinant proteins**

| Anti-HEV recombinant protein | ELISA with GST | Neutralization assay with HEV strain | |
|---|---|---|---|
| | | Burma | Mexico |
| Anti-pN309 | + | + | + |
| Anti-pN336 | + | + | + |
| Anti-pN364 | + | + | + |
| Anti-pN393 | + | + | + |
| Anti-pN421 | + | + | + |
| Anti-pN452 | + | + | + |
| Anti-pN499 | + | - | - |
| Anti-pC617 | + | + | + |
| Anti-pC580 | + | - | - |
| Anti-pC540 | + | - | - |
| Anti-pC507 | + | - | - |
| Anti-pB166 | + | + | + |

As shown in Table 4, all the serum samples were immunoreactive to GST in ELISA, indicating that the immunized mice generated antibodies to these truncated HEV-GST fusion proteins. The *in vitro* neutralization assay was then performed with both the HEV Burma and Mexico strains again. Surprisingly, the immune serum samples against the protein pN309, pN336, pN364, pN393, pN421, pN452, which were truncated from N-terminus of pF4 at position of aa 309, 336, 364, 393, 421, and 452 respectively, were similar to anti-pF4 in neutralizing activity to the both strains. However, when the immune serum sample against pN499, which was truncated from N-terminus of pF4 at position of aa 499, was applied, the neutralizing activity disappeared, suggesting that one or more amino acid residue in the region between aa 452 and aa 499 is significant to constitute the HEV neutralizing antigenic epitope(s).

For those serum samples against the protein pC617, pC580, pC540, pC507, which were truncated from C-terminus of pF4 at position of aa 617, 580, 540 and 507 respectively, only anti-pC617 neutralized the HEV strains in the *in vitro* neutralization assay. Thus, the amino acid residues between aa 617 and aa 660 at the extreme C-terminus of pORF2 are not essential to the neutralizing antigenic epitope(s) construction, but one or more amino acid residue in the region between aa 580 and aa 617 is significantly indispensable.

Finally, when the immune serum sample against the protein pB166, which was truncated from both N and C-terminus of pF4 at the position of aa 452 and 617, was performed in the *in vitro* neutralization assay, a very consistent result was obtained. This serum sample, anti-pB166, neutralized both the Burma and Mexico strains. Collectively, these data strongly indicated that the HEV neutralizing antigenic epitope(s) could be mapped within the sequence of aa 452-617 of pORF2 and be efficiently modeled with pB166, the 166 amino acid long recombinant protein.

### Cross-neutralization of the anti-pB166 to different genotypes or subtypes of HEV

As shown above, pB166 was the minimal protein that contained the HEV neutralizing antigenic epitope(s). It is reasonable that this protein is of the lowest nonspecific reactivity because of its shortest sequence. Although the immune serum sample against pB166 demonstrated neutralizing activity to both homologous Burma strain and heterogeneous Mexico strain, its cross-neutralization to other geographic HEV strains was tested. A quantitative cross-neutralization assay was performed with HEV strains derived from Burma, Pakistan, Morocco, Mexico, and USA, which represent different genotypes and subtypes of the HEV. The immune serum sample against pB166 was 2-fold diluted from 1:10 and mixed with 100 cell culture infectious doses of each HEV strain, respectively. After incubation at 37°C for 1 hour, the mixtures were inoculated to PLC/PRF/5 cell monolayers. Finally, the cross-neutralization was determined based on PCR detection as described in Materials and Methods. The neutralizing titers of anti-pB166 to different strains, except to Morocco strain, were not significantly variable as shown in Table 5.

**Table 5. Cross-neutralizing endpoint titration of anti-pB166 with different geographic HEV strains**

| HEV strain | Genotype | Subtype | Neutralizing titer |
|---|---|---|---|
| Burma | 1 | 1a | 1:640 |
| Pakistan | 1 | 1b | 1:1280 |
| Morocco | 1 | 1c | 1:20 |
| Mexico | 2 | | 1:640 |
| US | 3 | | 1:640 |

### REFERENCES

Emini E. A., J. V. Hughes, D. S. Perlow, and J. Boger. 1985. Induction of hepatitis A virus-neutralizing antibody by a virus-specific synthetic peptide. J. Virol. 55:836-839.

Khudyakov, Y. E., E. N. Lopareva, D. L. Jue, T. K. Crews, S. P. Thyagarajan, and H. A. Fields. 1999. Antigenic Domains of the open reading frame 2-encoded protein of hepatitis E virus. J. Clin. Microbiol. 37:2863-2871.

Kwo, P. Y., G. G. Schlauder, H. A. Carpenter, P. J. Murphy, J. E. Rosenblatt, G. J. Dawson, E. E. Mast, K. Krawczynski, and V. Balan.1997. Acute hepatitis E by a new isolate acquired in the United States. Mayo Clin. Proc. 72:1133-1136.

Meng, J-. H., P. Dubreuil, and J. Pillot. 1997. A new PCR-based seroneutralization assay in cell culture for diagnosis of hepatitis E. J. Clin. Microbiol. 35:1373-1377.

Meng, J-. H., J. Pillot, X. Dai, H. A. Fields, and Y. E. Khudyakov. 1998. Neutralization of different geographic strains of the hepatitis E virus with anti-hepatitis E virus-positive serum samples obtained from different sources. Virology 249:316-324.

Meng, X-. J., R. H. Purcell, P. G. Halbur, J. R. Lehman, D. M. Webb, T. S. Tsareva, J. S. Haynes, B. J. Thacker, and S. U. Emerson. 1997. A novel virus in swine is closely related to the human hepatitis E virus. Proc. Natl. Acad. Sci. USA 94:9860-9865.

Neurath, A. R., S. B. H. Kent, K. Parker, A. M. Prince, N. Strick, B. Brotman, and P. Sproul. 1986. Antibodies to a synthetic peptide from the preS 120-145 region of the hepatitis B virus envelope are virus-neutralizing. Vaccine 4:35-37.

Purdy, M. A., K. A. McCaustland, K. Krawczynski, J. Spelbring, G. R. Reyes, and D. W. Bradley. 1993. Preliminary evidence that a trpE-HEV fusion protein protects cynomolgus macaques against challenge with wild-type hepatitis E virus (HEV). J. Med. Virol. 41:90-94.

Purdy, M. A., K. A. McCaustland, K. Krawczynski, A. Tam, M. J. Beach, N. C. Tassopoulos, G. R. Reyes, and D. W. Bradley. 1992. Expression of a hepatitis E virus (HEV)-trpE fusion protein containing epitopes recognized by antibodies in sera from human cases and experimentally infected primates. Arch. Virol. 123:335-349.

Schlauder, G. G., G. J. Dawson, J. C. Erker, P. Y. Kwo, M. F. Knigge, D. L. Smalley, J. E. Rosenblatt, S. M. Desai, and I. K. Mushahwar.1998. The sequence and phylogenetic analysis of a novel hepatitis E virus isolated from a patient with acute hepatitis reported in the United States. J. Gen. Virol. 79:447-456.

Shimizu, Y. K., H. Igarashi, T. Kiyohara, T. Cabezon, P. Farci., R. H. Purcell, and H. Yoshikura. 1996. A hyperinzmune serum against a synthetic peptide corresponding to the hypervariable region 1 of hepatitis C virus can prevent viral infection in cell cultures. Virology 223:409-412.

Tam, A. W., M. M. Smith, M. E. Guerra, C-. C. Huang, D. W. Bradley, K. E. Fry, and G. R. Reyes. 1991. Hepatitis E virus (HEV): molecular cloning and sequencing of the full-length viral genome. Virology 185:120-131.

Tsarev, S. A., T. S. Tsareva, S. U. Emerson, S. Govindarajan, M. Shapiro, J. L. Gerin, and R. H. Purcell. 1994. Successful passive and active immunization of cynomolgus monkeys against hepatitis E. Proc. Natl. Acad. Sci. USA 91:10198-10202

Tsarev, S. A., T. S. Tsareva, S. U. Emerson, S. Govindarajan, M. Shapiro, J. L. Gerin, and R. H. Purcell. 1997. Recombinant vaccine against hepatitis E: dose response and protection against heterologous challenge. Vaccine 15:1834-1838.

Yarbough, P. O., K. Krawczynski, A. W. Tam, C. P. McAtee, K. A. McCaustland, Y. Zhang, N. Garcon, J. Spelbring, D. Carson, F. Myriam, J. D. Lifson, M. Slaoui, J. P. Prieels, H. Margolis, and T. R. Fuerst. 1997. Prevention of hepatitis E using r62K subunit vaccine: full protection against heterologous. HEV challenge in cynomolgus macaques, p. 650-655. *In* M. Rizzetto, R. H. Purcell, J. L. Gerin, and G. Verme (ed.), Viral hepatitis and liver disease. Edizioni Minerva Medica, Turin, Italy.

Yewdell, J., and J. Bennink. 1997. Immune responses to viruses, p. 271-305. In D. D. Richman, R. J. Whitley, and F. G. Hayden (ed.), Clinical virology. Churchill Livingstone, New York, NY.

### SEQUENCES

ORF2 and pORF2 (SEQ. ID NO.:1) (attached page)
YK-1291 (5'- GTT GTC TCA GCC AAT GGC GAG CC) (SEQ. ID NO.:2)
YK-1294 (5'- GCC TGC GCG CCG GTC GCA ACA) (SEQ. ID NO.:3)
YK-1292 (5'- TGG AGA ATG CTC AGC AGG ATA A) (SEQ. ID NO.: 4)
YK-1293 (5'- TAA GTG GAC TGG TCG TAC TCG GC) (SEQ. ID NO.:5)

## Claims

1. Use of a HEV polypeptide comprising at least 50 amino acid residues between amino acid residues 452 and 617 from the C-terminal of the HEV pORF2 protein, including at least one neutralizing epitope for preparing a composition to elicit neutralizing anti HEV antibodies.

2. Use according to claim 1 wherein the HEV polypeptide comprises at least about 100 amino acid residues between amino acid residues 452 and 617 from the C-terminal of the HEV pORF2 protein, including at least one neutralizing epitope.

3. Use according to claim 1 or claim 2 wherein the HEV polypeptide comprises the amino acid residues 452 through 617 of the C-terminal of the HEV pORF2 protein.

4. Use of claim 1 wherein the HEV polypeptide comprises amino acid residues from the C-terminal of the HEV pORF2 protein until aa 617 and containing at least amino acid residues 452 through 617.

5. Use of a polypeptide or an immunogenic molecule as defined in one of claim 1 to 4 for preparing a vaccine to induce a neutralizing immune response against HEV.

6. Use of an immunogenic molecule comprising a neutralizing HEV polypeptide comprising at least amino acid residues 452 through 617 from the C-terminal of the HEV pORF2 protein, or conservative modifications thereof for preparing a vaccine to induce a neutralizing immune response against HEV.

7. Use of a polypeptide of one of claims 1 to 5 for preparing a pharmaceutical composition.

8. A neutralizing HEV polypeptide consisting of one of the following amino acid sequences: amino acids 274 to 660; 309 to 660; 336 to 660; 364 to 660; 393 to 660; 421 to 660; 452 to 660; 274 to 617; and 452 to 617 from C-terminal of the HEV pORF2 protein or conservative modifications thereof.

9. Pharmaceutical composition comprising a polypeptide of claim 8.

10. An antibody elicited to one of the polypeptides of claim 8.

## Patentansprüche

1. Verwendung eines HEV-Polypeptids, das mindestens 50 Aminosäurereste von den Aminosäureresten 452 bis 617 vom C-Ende des HEV-pORF2-Proteins aufweist einschließlich mindestens eines neutralisierenden Epitops zur Herstellung einer Zusammensetzung, um neutralisierende Anti-HEV-Antikörper zu erzeugen.

2. Verwendung nach Anspruch 1, wobei das HEV-Polypeptid mindestens etwa 100 Aminosäurereste von den Aminosäureresten 452 bis 617 vom C-Ende des HEV-pORF2-Proteins aufweist, einschließlich mindestens eines neutralisierenden Epitops.

3. Verwendung nach Anspruch 1 oder Anspruch 2, wobei das HEV-Polypeptid die Aminosäurereste 452 bis 617 des C-Endes des HEV-pORF2-Proteins aufweist.

4. Verwendung nach Anspruch 1, wobei das HEV-Polypeptid Aminosäurereste vom C-Ende des HEV-pORF2-Proteins bis aa 617 aufweist und mindestens die Aminosäurereste 452 bis 617 enthält.

5. Verwendung eines Polypeptids oder eines immunogenen Moleküls, wie in einem der Ansprüche 1 bis 4 definiert, zur Herstellung eines Impfstoffs, um eine neutralisierende Immunantwort gegen HEV hervorzurufen.

6. Verwendung eines immunogenen Moleküls, das ein neutralisierendes HEV-Polypeptid aufweist, das mindestens Aminosäurereste 452 bis 617 vom C-Ende des HEV-pORF2-Proteins aufweist, oder konservative Modifikationen davon, zur Herstellung eines Impfstoffs, um eine neutralisierende Immunantwort gegen HEV zu induzieren.

7. Verwendung eines Polypeptids nach einem der Ansprüche 1 bis 5 zur Herstellung einer pharmazeutischen Zusammensetzung.

8. Neutralisierendes HEV-Polypeptid bestehend aus einer der folgenden Aminosäuresequenzen: Aminosäuren 274 bis 660; 309 bis 660; 336 bis 660; 364 bis 660; 393 bis 660; 421 bis 660; 452 bis 660; 274 bis 617; und 452 bis 617 vom C-Ende des HEV-pORF2-Proteins oder konservative Modifikationen davon.

9. Pharmazeutische Zusammensetzung enthaltend ein Polypeptid nach Anspruch 8.

10. Antikörper, der gegen eines der Polypeptide von Anspruch 8 erzeugt wurde.

## Revendications

1. Utilisation d'un polypeptide de virus HEV comprenant au moins 50 résidus d'acides aminés entre les résidus d'acides aminés 452 et 617 provenant de la terminaison C de la protéine pORF2 du virus HEV, comprenant au moins un épitope de neutralisation pour préparer une composition pour provoquer des anticorps anti virus HEV de neutralisation.

2. Utilisation selon la revendication 1, dans laquelle le polypeptide de virus HEV comprend au moins environ 100 résidus d'acides aminés entre les résidus d'acides aminés 452 et 617 à partir de la terminaison C de la protéine pORF2 du virus HEV, comprenant au moins un épitope de neutralisation.

3. Utilisation selon la revendication 1 ou la revendication 2, dans laquelle le polypeptide de virus HEV comprend les résidus d'acides aminés 452 à 617 de la terminaison C de la protéine pORF2 du virus HEV.

4. Utilisation selon la revendication 1, dans laquelle le polypeptide de virus HEV comprend des résidus d'acides aminés provenant de la terminaison C de la protéine pORF2 du virus HEV jusqu'à aa 617 et contenant au moins les résidus d'acides aminés 452 à 617.

5. Utilisation d'un polypeptide ou d'une molécule immunogène selon l'une des revendications 1 à 4, pour préparer un vaccin afin d'induire une réponse immunitaire de neutralisation contre le virus HEV.

6. Utilisation d'une molécule immunogène comprenant un polypeptide de neutralisation du virus HEV comprenant au moins les résidus d'acides aminés 452 à 617 à partir de la terminaison C de la protéine pORF2 du virus HEV ou des modifications conservatrices de celle-ci pour préparer un vaccin destiné à induire une réponse immunitaire de neutralisation contre le virus HEV.

7. Utilisation d'un polypeptide selon l'une des revendications 1 à 5 pour préparer une composition pharmaceutique.

8. Polypeptide neutralisant le virus HEV constitué des séquences d'acides aminés suivantes : acides aminés 274 à 660 ; 309 à 660 ; 336 à 660 ; 364 à 660 ; 393 à 660 ; 421 à 660 ; 452 à 660 ; 274 à 617 ; et 452 à 617 à partir de la terminaison C de la protéine pORF2 du virus HEV ou de modifications conservatrices de celle-ci.

9. Composition pharmaceutique comprenant un polypeptide selon la revendication 8.

10. Anticorps produit pour l'un des polypeptides de la revendication 8.


### SEQUENCE LISTING

<110> JIHONG MENG
   HOWARD A. FIELDS
   YURI E. KHUDYAKOV
<120> NEUTRALIZING IMMUNOGENIC HEPATITIS E VIRUS POLYPEPTIDES (HEV)
<130> 14114.0340P1
<140> 60/195,380
   <141> 2000-04-07
<160> 5
<170> FastSEQ for Windows Version 4.0
<210> 1
   <211> 660
   <212> PRT
   <213> Artificial Sequence
<220>
   <223> Description of Artificial Sequence:/ Note = Synthetic construct
<400> 1
   Met Arg Pro Arg Pro Ile Leu Leu Leu Leu Leu Met Phe Leu Pro Val
   1 5 10 15
   Met Leu Ala Pro Pro Pro Gly Gln Pro Ser Gly Arg Arg Arg Gly Arg
   20 25 30
   Arg Ser Gly Gly Ser Gly Gly Gly Phe Trp Gly Asp Arg Val Asp Ser
   35 40 45
   Gln Pro Phe Ala Ile Pro Tyr Ile His Pro Thr Asn Pro Phe Ala Pro
   50 55 60
   Asp Val Thr Ala Ala Ala Gly Ala Gly Pro Arg Val Arg Gln Pro Ala
   65 70 75 80
   Arg Pro Leu Gly Ser Ala Trp Arg Asp Gln Ala Gln Arg Pro Ala
   Val
                       85                  90                  95
   Ala Ser Arg Arg Arg Pro Thr Thr Ala Gly Ala Ala Pro Leu Thr
   Ala
                   100                 105                 110
   Val Ala Pro Ala His Asp Thr Pro Pro Val Pro Asp Val Asp Ser
   Arg
               115                 120                 125
   Gly Ala Ile Leu Arg Arg Gln Tyr Asn Leu Ser Thr Ser Pro Leu
   Thr
           130                 135                 140
   Ser Ser Val Ala Thr Gly Thr Asn Leu Val Leu Tyr Ala Ala Pro
   Leu
   145                 150                 155
   160
   Ser Pro Leu Leu Pro Leu Gln Asp Gly Thr Asn Thr His Ile Met
   Ala
                       165                 170                 175
   Thr Glu Ala Ser Asn Tyr Ala Gln Tyr Arg Val Ala Arg Ala Thr
   Ile
                   180                 185                 190
   Arg Tyr Arg Pro Leu Val Pro Asn Ala Val Gly Gly Tyr Ala Ile
   Ser
               195                 200                 205
   Ile Ser Phe Trp Pro Gln Thr Thr Thr Thr Pro Thr Ser Val Asp
   Met
           210                 215                 220
   Asn Ser Ile Thr Ser Thr Asp Val Arg Ile Leu Val Gln Pro Gly
   Ile
   225                 230                 235
   240
   Ala Ser Glu Leu Val Ile Pro Ser Glu Arg Leu His Tyr Arg Asn
   Gln
                       245                 250                 255
   Gly Trp Arg Ser Val Glu Thr Ser Gly Val Ala Glu Glu Glu Ala
   Thr
                   260                 265                 270
   Ser Gly Leu Val Met Leu Cys Ile His Gly Ser Leu Val Asn Ser
   Tyr
               275                 280                 285
   Thr Asn Thr Pro Tyr Thr Gly Ala Leu Gly Leu Leu Asp Phe Ala
   Leu
           290                 295                 300
   Glu Leu Glu Phe Arg Asn Leu Thr Pro Gly Asn Thr Asn Thr Arg
   Val
   305 310 315
   320
   Ser Arg Tyr Ser Ser Thr Ala Arg His Arg Leu Arg Arg Gly Ala
   Asp
   325 330 335
   Gly Thr Ala Glu Leu Thr Thr Thr Ala Ala Thr Arg Phe Met Lys
   Asp
   340 345 350
   Leu Tyr Phe Thr Ser Thr Asn Gly Val Gly Glu Ile Gly Arg Gly
   Ile
   355 360 365
   Ala Leu Thr Leu Phe Asn Leu Ala Asp Thr Leu Leu Gly Gly Leu
   Pro
   370 375 380
   Thr Glu Leu Ile Ser Ser Ala Gly Cys Gln Leu Phe Tyr Ser Arg
   Pro
   385 390 395
   400
   Val Val Ser Ala Asn Gly Glu Pro Thr Val Lys Leu Tyr Thr Ser
   Val
   405 410 415
   Glu Asn Ala Gln Gln Asp Lys Gly Ile Ala Ile Pro His Asp Ile
   Asp
   420 425 430
   Leu Gly Glu Ser Arg Val Val Ile Gln Asp Tyr Asp Asn Gln His
   Glu
   435 440 445
   Gln Asp Arg Pro Thr Pro Ser Pro Ala Pro Ser Arg Pro Phe Ser
   Val
   450 455 460
   Leu Arg Ala Asn Asp Val Leu Trp Leu Ser Leu Thr Ala Ala Glu
   Tyr
   465 470 475
   480
   Asp Gln Ser Thr Tyr Gly Ser Ser Thr Gly Pro Val Tyr Val Ser
   Asp
   485 490 495
   Ser Val Thr Leu Val Asn Val Ala Thr Gly Ala Gln Ala Val Ala
   Arg
   500 505 510
   Ser Leu Asp Trp Thr Lys Val Thr Leu Asp Gly Arg Pro Leu Ser
   Thr
   515 520 525
   Ile Gln Gln Tyr Ser Lys Thr Phe Phe Val Leu Pro Leu Arg Gly Lys
   530 535 540
   Leu Ser Phe Trp Glu Ala Gly Thr Thr Lys Ala Gly Tyr Pro Tyr Asn
   545 550 555 560
   Tyr Asn Thr Thr Ala Ser Asp Gln Leu Leu Val Glu Asn Ala Ala Gly
   565 570 575
   His Arg Val Ala Ile Ser Thr Tyr Thr Thr Ser Leu Gly Ala Gly Pro
   580 585 590
   Val Ser Ile Ser Ala Val Ala Val Leu Ala Pro His Ser Ala Leu Ala
   595 600 605
   Leu Leu Glu Asp Thr Leu Asp Tyr Pro Ala Arg Ala His Thr Phe Asp
   610 615 620
   Asp Phe Cys Pro Glu Cys Arg Pro Leu Gly Leu Gln Gly Cys Ala Phe
   625 630 635 640
   Gln Ser Thr Val Ala Glu Leu Gln Arg Leu Lys Met Lys Val Gly Lys
   645 650 655
   Thr Arg Glu Leu
   660
<210> 2
   <211> 23
   <212> DNA
   <213> Artificial Sequence
<220>
   <223> Description of Artificial Sequence:/ Note = Synthetic construct
<400> 2
   gttgtctcag ccaatggcga gcc 23
<210> 3
   <211> 21
   <212> DNA
   <213> Artificial Sequence
<220>
   <223> Description of Artificial Sequence:/ Note = Synthetic construct
<400> 3
   gcctgcgcgc cggtcgcaac a
   21
<210> 4
   <211> 22
   <212> DNA
   <213> Artificial Sequence
<220>
   <223> Description of Artificial Sequence:/ Note = Synthetic construct
<400> 4
   tggagaatgc tcagcaggat aa
   22
<210> 5
   <211> 23
   <212> DNA
   <213> Artificial Sequence
<220>
   <223> Description of Artificial Sequence:/ Note = Synthetic construct
<400> 5
   taagtggact ggtcgtactc ggc
   23